# EUROPEAN PATENT APPLICATION

(11) **EP 1 374 946 A1**
(43) Date of publication of application: **02.01.2004**
(21) Application number: 03022037.0
(22) Date of filing: 27.01.1999
(51) Int. Cl.: A61N 1/36, A61N 1/08

(54) **Muscle electrostimulator conformed in such a way as to avoid sensations of pain and reddening of the skin**

(30) Priority: 29.01.1998 IT PS980002
(62) Divisional of application: 99900593.7
(71) Applicant: Baldi Holding S.A., 1118 Luxembourg (LU)
(72) Inventor: Agabiti, Gabriele, 61100 Pesaro (IT); Corsi, Dario, 61100 Pesaro (IT); Ponsele', Michele, 61100 Pesaro (IT)
(74) Representative: Lanzoni, Luciano

(57) **Abstract**

The present invention relates to a muscle electrostimulator able to improve the physical training of athletes, suitable for use in rehabilitation therapy in general and in particular when it is impossible to perform rehabilitation exercises with various types of equipment, of for use in aesthetic treatments.

In contrast with the muscle electrostimulators currently in use, the one described appropriately limits the possible excess voltage which may occur on the electrodes in the case of accidental detachment thereof, thus avoiding sensations of pain in the person undergoing the treatment.

Furthermore, it avoids excessive heating and undesirable reddening or burns to the skin.

## Description

The present invention relates to a muscle stimulator able to improve the physical training of athletes, suitable for use in physical therapy in general and in particular when it is impossible to perform rehabilitation exercises with various types of equipment, or for use in aesthetic treatments.

In humans, muscle contraction takes place thanks to nerve stimuli sent voluntarily from the brain (central nervous system), which pass through the spinal cord and then through the motor nerves which innervate the various skeletal striated muscles in the body.

These muscles can also be stimulated to contract by external stimuli, such as that of an electrical shock.

Muscle electrostimulation is the practice which allows to obtain contractions of the skeletal striated muscles that are similar in every way to voluntary muscle contractions. This non-invasive practice, which can substitute the nerve signal sent by the central nervous system, can be utilised to develop the muscle characteristics of athletes, or in rehabilitation treatment for patients suffering from muscle atrophy due to the protracted lack of use of a set of muscles.

Electrostimulators generate electrical pulses which are sent to electrodes applied to the skin of the body parts to be treated. The way in which the pulses are used differs according to the individual, the type of muscle to be stimulated, and the characteristics of the skin to which the electrodes are to be applied.

The muscle electrostimulators currently in use have the drawback of supplying excess voltage which, in the case of accidental detachment of an electrode, can cause sensations of pain. This is because the breaking of the circuit, and the resulting increase in impedance towards infinite values, causes a sudden rise in the voltage supplied to the electrode by the generator, which attempts to maintain the current constant.

Another drawback of the devices currently in use is that of causing, in certain cases, the reddening of, or burns to, the skin near the electrodes.

The present invention has the purpose of allowing the effective electrostimulation of the various muscles of the body, avoiding sensations of the pain should an electrode become detached and avoiding the reddening or burning of the skin.

For this purpose, a monitoring circuit is used to control excess voltage on the electrodes by blocking the passage of current within an extremely brief time, during which the person is unable to feel any pain. By correctly repositioning the device, the emission of pulses is restored, with a voltage output which, in a ramped or in any case rising manner, increases from zero to the predetermined value, in such a way as not to cause any painful shocks.

By means of automatic temperature monitoring on the contact surface of the electrodes, the subject device also prevents excessive heating of the body part concerned and therefore the undesired reddening or burning of the skin. This allows the flow of current along the electrodes themselves to be limited or blocked in the case of an excessive rise in the temperature of the area whereto they are applied.

The invention shall now be described in detail, with reference to the accompanying plates and drawings, which illustrate a possible embodiment provided purely by way of example.
- Figure 1, plate 1 shows a block diagram of the device.
- Figure 2, plate 2 shows a possible version of the corresponding wiring diagram.

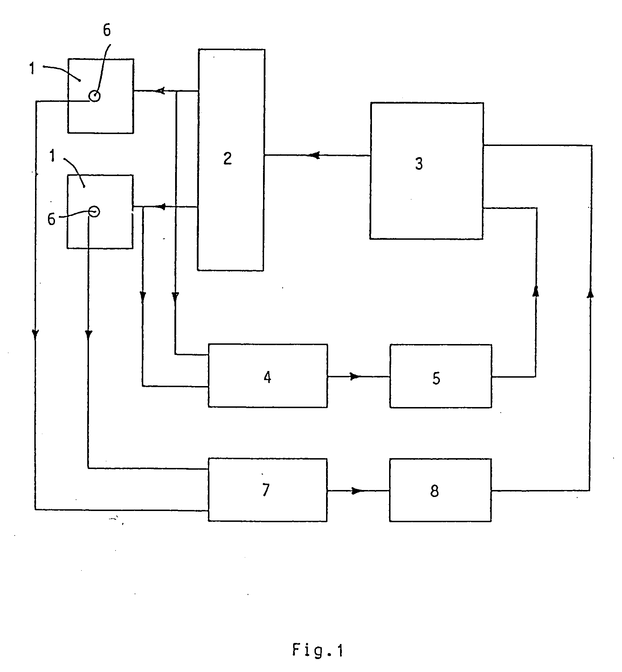

In Figure 1, the electrodes to be applied in variable number on the body parts involved are indicated with the reference number 1. These electrodes are connected to the pulse generator 2, which supplies electrical pulses whose characteristics are monitored by the central processing unit (CPU) 3. The amplitude of the pulses sent to the electrodes is monitored with the peak detector 4, whose output is converted from analogue to digital form by means of the analogue to digital converter 5. The digital input of this converted, proportional to the amplitude of the pulses sent to the electrodes, is read by the CPU 3 which, based on this amplitude, acts on the pulse generator 2, in such a way as to block current output within 5 microseconds in the case of temporary excess voltage due to the accidental detachment of an electrode. When the electrode is repositioned correctly, the CPU restores pulse emission, ramping up the amplitude from zero to the maximum value set previously by means of potentiometers. In this way, there is no need to set voltage to zero manually by means of the regulating potentiometers, which thus remain positioned on the pre-selected optimal value, avoiding time wastage and the possibility of errors.

The number 6 indicates the temperature sensors applied inside the electrodes 1. The output signals of the sensors are amplified by amplifier 7 and converted to a digital signal by the analogue-to-digital converter 8. The CPU reads this output and, in case of an excessive temperature increase, it limits or blocks the pulses sent by the electrodes.

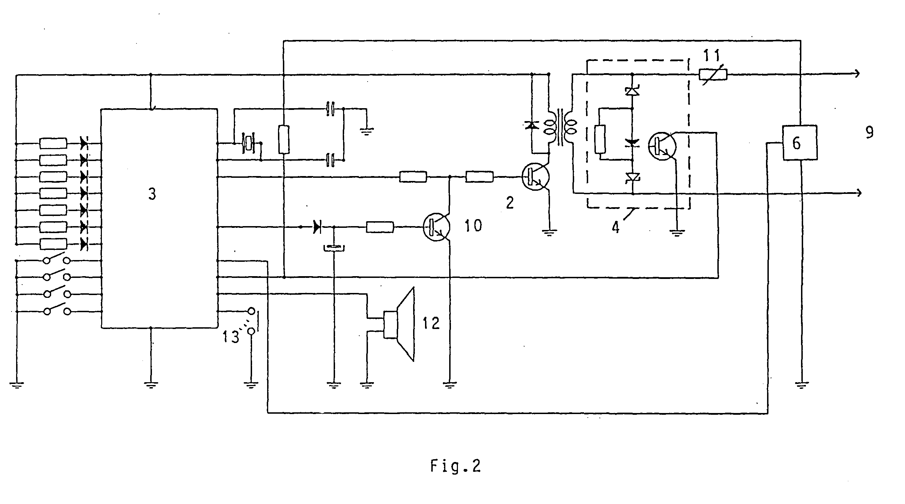

In Figure 2, the electrode terminals are indicated with the number 9. In this figure the same reference numbers as in Figure 1 are used to indicate the pulse generator 2, the CPU 3, the peak detector 4 and the temperature sensor 6. The analogue-to-digital converters and the amplifier are integrated into the CPU (but could obviously be separated). The ramp generator is indicated with the number 10 and the potentiometer for the regulation of the current sent to the electrode is indicated with the number 11. The acoustic alarm is indicated with the number 12 and the start/reset button is indicated with the number 13.

Obviously, the number of electrodes and the constructive details of the circuit could differ from those illustrated solely by way of example, without thereby departing from the scope of the present invention.

## Claims

1. Muscle electrostimulator conformed in such a way as to avoid sensations of pain and reddening of the skin, comprising an impulse generator (2), monitoring and input circuits, electrodes (1), regulating components and a circuit (4, 5, 3) for monitoring excess voltage on the electrodes (1) which, if the prescribed limit is exceeded, blocks the output of current within a time of such a duration that the person is unable to perceive any pain, **characterized in that** it further comprises a circuit (7,8,3) that automatically monitors the temperature on the surface in contact with the electrodes (1) and limits or blocks the flow of current in case of an increase in skin temperature which is predetermined so as to prevent undesired reddening or burning of the skin itself due to excessive heating of the body part concerned.

2. Electrostimulator according to claim 1, **characterised in that**, it comprises means (10) for reastoring the emission of the pulses after the electrode is correctly repositioned, said means (10) for reastoring the emission of the pulses acting with a voltage that ramps up, or in any case rises, from zero to the pre-selected value, which can be adjusted as desired, in such a way as to prevent painful shocks.

3. Electrostimulator according to claim 2, **characterised in that** it comprises a CPU (3), said means (10) for restoring the emission of the pulses being automatically activated by the CPU (3) after correct repositioning of the electrode.
